# EUROPEAN PATENT SPECIFICATION

(11) **EP 2 181 149 B1**
(45) Date of publication and mention of the grant of the patent: **20.07.2016**
(21) Application number: 08782398.5
(22) Date of filing: 25.07.2008
(51) Int. Cl.: C08K 5/10, C03C 27/00, C07C 69/78

(54) **NOVEL HIGH VISCOSITY LIQUID BENZOATE ESTER COMPOSITIONS AND POLYMER COMPOSITIONS CONTAINING SAID ESTER COMPOSITIONS**
NEUE HOCHVISKOSE FLÜSSIGE BENZOATESTERZUSAMMENSETZUNGEN UND POLYMERZUSAMMENSETZUNGEN, DIE DIE ESTERZUSAMMENSETZUNGEN ENTHALTEN
NOUVELLES COMPOSITIONS D'ESTER DE BENZOATE LIQUIDES À VISCOSITÉ ÉLEVÉE ET COMPOSITIONS DE POLYMÈRE CONTENANT LES COMPOSITIONS D'ESTER

(30) Priority: 30.07.2007 US 830366
(43) Date of publication of application: 05.05.2010
(73) Proprietor: Eastman Specialties Holdings Corporation, 37660 Sullivan County (US)
(72) Inventor: JOSHI, Makarand, Grayslake, IL 60030 (US); ARENDT, William, D., Libertyville, IL 60042 (US); STREPKA, Arron, M., Lagrange Park, IL 60526 (US); SCHROEDER, Joel, Racine, WI 53405 (US)
(74) Representative: Ricker, Mathias
(86) International application number: PCT/US2008/071193
(87) International publication number: WO 2009/018151

(56) References cited:
- EP-B1- 0 673 916
- US-A- 4 173 557
- US-A- 5 006 585
- US-A- 5 039 728
- US-A- 5 676 742
- US-A1- 2003 092 808
- US-A1- 2003 109 632
- US-B2- 6 919 397
- DATABASE WPI Week 199214 Thomson Scientific, London, GB; AN 1992-111155 XP002688824, -& JP 4 055481 A (KYOWA YUKA KK) 24 February 1992 (1992-02-24)
- BROOKFIELD: 'MORE SOLUTIONS TO STICKY PROBLEMS A Guide to Getting More From Your Brookfield Viscometer' BROOKFIELD, [Online] 01 December 2005, pages 1 - 55, XP055064554 Retrieved from the Internet: <URL:http://www.brookfieldengineering.com/d ownload/files/more_solutions.pdf> [retrieved on 2013-05-29]
- "ASTM D2196 - 10 Standard Test Methods for Rheological Properties of Non-Newtonian Materials by Rotational (Brookfield type) Viscometer", ASTM INTERNATIONAL STANDARD, ASTM INTERNATIONAL, US, 1 January 2010 (2010-01-01), pages 236-240, XP009160351,

## Description

### FIELD OF THE INVENTION

This invention relates to benzoate ester compositions. More particularly, this invention relates to the benzoates of a particular branched diol. This benzoate composition exhibits an unexpectedly high viscosity, making it particularly unique and useful as a plasticizer or other type of additive for one-and two part polymer compositions.

This invention also relates to one- and two-part polymer compositions containing one of the present benzoate ester compositions as a plasticizer.

### BACKGROUND OF THE INVENTION

### Description of the Prior Art

Esters of both aliphatic and aromatic carboxylic acids with mono-and polyfunctional alcohols are widely used as plasticizers and other types of additives for a variety of polymers. The viscosities of liquid benzoic acid esters derived from 1) mono- and difunctional alcohols, 2) glycols containing from 2 to 4 carbon atoms or 3)oligomers of these glycols are typically no higher than about 150 centipoises at 25° C., measured using a Brookfield LVT viscometer. Unless otherwise specified all viscosity values in this specification and the accompanying claims refer to values measured at 25° C. using this viscometer.

All of the aforementioned benzoic acid esters are employed as plasticizers and other types of modifiers and additives in a variety of organic polymer compositions.

The viscosities of liquid compositions containing both the mono-and dibenzoates of either 1) a diol or a glycol such as ethylene and propylene glycols or 2) an oligomeric ethylene or propylene glycol typically do not increase substantially as the relative concentration of the dibenzoate is increased up to 100 weight percent. The viscosities of these ester compositions are typically less than 150 centipoises.

Some monomeric phthalic acid esters derived from a) monofunctional alcohols and b) half esters and glycols containing from 2 up to about 8 carbon atoms typically exhibit viscosities of up to about 1200 centipoise, however health and/or environmental regulations limit the use of phthalates in certain end use applications.

A commercially available product referred to in the literature as the dibenzoate of 2,2,4-trimethyl-1,3-pentanediol, hereinafter referred to as TMPDB, is actually a mixture of this ester with monobenzoate and degradation products. The viscosity of this mixture is about 400 centipoise.

The present invention is based on the discovery that as the percent by weight of TMPDB in this commercially available product is increased from 85 to 100 percent its viscosity increases dramatically, reaching about 1200 centipoise. Mixtures containing more than 85 weight percent of the dibenzoate have not heretofore been disclosed and are considered novel.

### SUMMARY OF THE INVENTION

This invention provides a liquid benzoate ester composition comprising the general formula 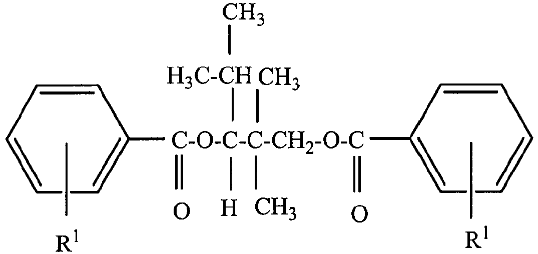 and at least one monobenzoate of a formula selected from the group consisting of 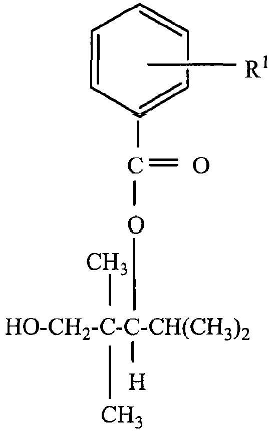 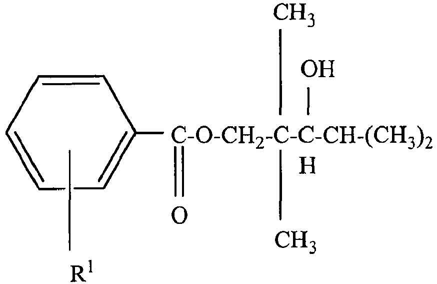 and mixtures thereof, wherein R¹ is alkyl or H, and mixtures thereof, and the concentration of the dibenzoate is effective for imparting the liquid benzoate ester composition with a viscosity of from about 425 to about 1200 centipoise, measured at 25°C using a Brookfield LVT viscometer, to said composition, wherein the concentration of said diester is above 87 weight percent of the benzoate ester composition.

The concentration of dibenzoate is effective for providing a viscosity of about 550 to about 1200 centipoise, measured at 25°C using a Brookfield LVT viscometer, to said composition. In an important aspect, the dibenzoate is from about 87 to about 100 weight percent of the composition. In another aspect, the dibenzoate ester composition is provided with from about 5 to about 95 weight percent of an additional liquid or solid. Liquid and solid components may include polymers, other plasticizers, liquid carriers, solvents, pigments, fillers, catalysts, curing agents, adhesion promoters, cure retarders and deaerators.

In another aspect, the benzoate ester composition includes 5 weight percent or less, based on the total weight of the benzoate ester composition, of monobenzoate. The benzoate ester composition can also contain 5 weight percent or less, based on the total weight of the benzoate ester composition, of unesterified diol, such as for example 2,2,4-trimethyl-1,3-pentanediol (HOCH₂C(CH₃)₂CH(OH)CH(CH₃)CH₃). In the aspect where the benzoate ester composition includes an unesterified diol, the viscosity of the benzoate ester composition will be at least about 900 centipose.

This invention also provides one- and two-part polymer compositions containing an ester composition of this invention as a plasticizer.

In this aspect, a plasticized polymer composition is provided that includes the liquid benzoate ester and at least one organic polymer. Organic polymers that may be utilized include polysulfides, homopolymers and copolymers of vinyl chloride, acrylic polymers, polyesters, polyurethanes, elastomeric copolymers of styrene, and mixtures thereof. The liquid benzoate ester may be from about 5 weight percent to about 95 weight percent of the plasticized polymer composition.

In another aspect, a two-part curable polymer composition is provided that includes a cross-linkable polymer and at least one plasticizer as a first part and the liquid benzoate ester as a second part. The second part may also include a catalyst for cross-linking the cross-linkable polymer. Cross-linkable polymers that may be used include polysulfides, polyurethanes, acrylic polymers, and mixtures thereof. The plasticizer may include esters of benzoic acid and monohydric alcohols, esters of benzoic acid and diols, esters of benzoic acid with at least one glycol containing from 2 to 4 carbon atoms, esters of benzoic acid with oligomers of said glycol, and mixtures thereof.

### DETAILED DESCRIPTION OF THE INVENTION

The uniqueness of the present benzoate ester compositions resides in their unexpectedly high viscosity relative to other non-polymeric esters of benzoic acid, including the aforementioned commercially available product identified as the dibenzoate of 2,2,4-trimethyl-1,3-pentanediol. The viscosity of the commercially available product is typically 400 centipoise at 25° C and the hydroxyl number, a measure of the monobenzoate content, is about 12.

The viscosities of the present ester compositions are directly proportional to the dibenzoate concentration. What distinguishes the present ester compositions from other mixtures of mono- and dibenzoates is not only the magnitude of the viscosity of these compositions, but also the unexpectedly large percentage increase in viscosity observed at dibenzoate concentrations above about 87 weight percent.

The ester compositions of this invention can be prepared using conventional methods for preparing other esters of benzoic acid. In accordance with this method the esterification reaction is continued until a conversion to diester of greater than 87 percent is achieved. It is at this level that the greatest percentage increase in viscosity is observed. Methods for achieving this level of dibenzoate content and for determining the extent of conversion to dibenzoate by measuring the concentration of unreacted carboxylic acid and hydroxyl groups in a reaction mixture are known to those skilled in the art.

Substituted benzoic acids such as toluic acid can be used in place of benzoic acid.

Suitable catalysts for the esterification reaction include but are not limited to compounds of the elements in groups IVA and IVB of the periodic table.

Methods for isolating and purifying mixtures of mono- and dibenzoates from reaction mixtures used to prepare them are known in the art and are not part of the present invention.

The unexpectedly high viscosity of the present dibenzoate compositions make them particularly useful plasticizers in two part polymer compositions containing a cross-linkable polymer in one part and a cross-linking agent and/or a catalyst in a second part. Including a high viscosity benzoate ester composition of the present invention as a plasticizer in the second part of these polymer compositions increases the viscosity of this part to a level comparable to that of the first part containing the polymer, thereby facilitating blending of the two parts to form a homogeneous curable composition.

In the past increasing the viscosity of the non-polymer portion of a 2-part composition was typically achieved using high viscosity phthalic acid esters of monohydric alcohols or by adding thickening agents, fillers, or other viscosity modifiers that could adversely affect the properties of the cured polymer. A disadvantage of phthalates is that they either cannot be used in certain applications or require warning labels.

The high viscosities of the plasticizers of this invention reduces separation of these plasticizers from other components, resulting in increased storage stability of the compositions. Storage stability is the lack of settling or separation of the solid and/or liquid components of the composition.

Polymers particularly suitable for use with the high viscosity benzoate ester compositions of this invention include but are not limited to polysulfides, homo- and copolymers of vinyl chloride, polyesters, polyurethanes, acrylic polymers, epoxide polymers and elastomeric polymers including but not limited to those derived from styrene.

The present benzoate ester compositions can also be used in the same manner as other esters of benzoic acid, namely 1) as plasticizers, viscosity modifiers, and other types of additives and carriers in non-aqueous polymer compositions, 2) in aqueous adhesives containing polyvinyl esters and 3) to adjust the viscosity of curable and foamable polymer compositions.

Uses of polymer compositions containing the benzoate esters of the present invention include but are not limited to paints, coatings, adhesives, construction and automotive sealants, including insulating glass sealants, inks, elastomers, foams and films.

The benzoate ester compositions of this invention can be used in combination with known benzoate esters to achieve desired properties in specific polymer compositions. For example, it has now been found that when the benzoate ester composition of this invention is blended with glycol benzoate(s) such as dipropylene glycol dibenzoate the resultant polymer composition becomes softer and more pliable. This is particularly desirable when the polymer composition is intended for use as a sealant. The benzoate ester composition and glycol benzoates are blended in an amount effective for providing a depression of Shore A Hardness of about 15 to about 22%, an increase in % elongation of about 33 to about 140%, and a depression of Break Stress of about 9 to about 20%. In this aspect, the benzoate ester composition may be blended with up to an equal amount of the glycol benzoate.

The following examples describe the preparation of a preferred benzoate ester composition of this invention and the performance of this composition as a plasticizer in the non-polymer portion of a two-part curable polysulfide composition.

Unless otherwise specified all parts and percentages are by weight and properties were measured at 25° C. Viscosities were measured using a Brookfield model LVT viscometer.

### Example 1

This example describes the preparation of a preferred benzoate ester composition of this invention.

An esterification reaction mixture was prepared by blending benzoic acid, 2,2,4-trimethyl-1,3-pentanediol and an effective amount of a suitable esterification catalyst in a reactor typical of those used for esterification reactions. The reactor included means for measuring the temperature of the reaction mixture and removing the water formed as a by-product of the reaction. The molar ratio of acid to diol was 2.2:1.0

The resultant mixture was heated with stirring to a temperature sufficient to distill the water formed as a by-product of the esterification reaction. Heating was continued until OH number of the reaction mixture was 8. The reaction mixture was purified using known techniques to isolate the desired ester composition.

The viscosity of the final ester composition was 900 centipoise.

### Example 2

This example demonstrates the use of the benzoate ester described in Example 1 as the replacement for a high-viscosity phthalate ester plasticizer in the non-polymer portion of a 2-part curable polysulfide composition.

Part A of the composition was prepared using the following ingredients:
100 parts of a liquid polysulfide polymer available as Thiopolast® G 21 from Akzo Nobel
35 parts of one of four different plasticizers: butyl benzyl phthalate, referred to hereinafter as A1; dipropylene glycol dibenzoate, referred to hereinafter as A2 (Benzoflex^{®} 9-88); a 6.6 : 17.4 : 4.3 weight ratio mixture of dipropylene glycol dibenzoate : diethylene glycol dibenzoate : triethylene glycol dibenzoate, referred to hereinafter as A3 (Benzoflex 2088); and propylene glycol dibenzoate, referred to hereinafter as A4 (Benzoflex 284).
1.2 parts of gamma-glycodioxypropyl trimethoxy silane
155 parts of ground calcium carbonate and
32.5 parts of precipitated calcium carbonate

Procedure for preparing part A:
All of the liquid ingredients were blended to homogeneity;
Ground calcium carbonate was added to liquid ingredients and blended in;
One half of precipitated calcium carbonate was added and blended in;
Remaining precipitated calcium carbonate was blended in;
Resulting mixture blended sufficiently to achieve a rating of 4 using a Hegmann grind gauge.

Part B was prepared using the following ingredients:
100 parts of one of two different plasiticizers;
64.9 parts of manganese (IV) oxide;
21 parts of carbon black
7.1 parts of 1,1,3,3-tetramethyl guanidine

The two plasticizers evaluated in part B were:
The benzoate ester of Example 1, referred to hereinafter as B 1;
as B2, 2,2,4-trimethyl-1,3-pentanediol isobutyrate(1) benzylphthalate(3),
referred to hereinafter as TMPDIBBP, exhibiting the formula CH₃CH(CH₃)C(O)OCH₂C(CH₃)₂CHXCH(CH₃)₂
X = -OC(O)PhC(O)OCH₂C₆H₅
B2 was evaluated for comparative purposes.

Part B was prepared using the following procedure:
40 percent of the plasticizer being evaluated was blended with the remaining ingredients to homogeneity;
the resultant mixture was ground using a 3-roll mill with a 0.10 mm gap to a rating of between 4 and 5 on the Hegmann grind gauge;
the remainder of the plasticizer was added and the composition blended to homogeneity.

Curable compositions were prepared by blending parts A and B in a 10:1 volumetric ratio. The resultant mixtures were degassed by passing them through a 3-roll mill with a gap of 0.2 mm. Test samples were prepared for the evaluation of tensile properties and hardness (durometer measurement) by placing the resultant mixtures into polyethylene terephthalate molds measuring 5 inches (12.7 cm.) in length, ¾ inch (1.9 cm.) in width and 1/16 inch (0.16 cm) in depth. Five samples were prepared from each of the compositions.

The test samples were cured for 3 days under ambient conditions followed by 4 hours at 70° C., following which the samples were allowed to cool for 4 hours under ambient conditions. The thickness of each sample was measured following which the hardness of the samples was determined using ASTM test procedure D2240 and the tensile strengths measured using ASTM test procedure D-638.

**TABLE 1**

| Plasticzers | Shore A Hardness | | Tensile Strength | |
|---|---|---|---|---|
| | Initial | 10 Second | % Elongation | Break Stress (psi) |
| A1, B2* | 40 | 40 | 49 | 136 |
| A2, B1 | 36 | 34 | 96 | 124 |
| A3, B1 | 44 | 42 | 40 | 143 |
| A4, B1 | 46 | 44 | 72 | 155 |

| | | | | |
|---|---|---|---|---|
| * - Evaluated for comparative purposes | | | | |

The data in Table 1 indicate that the combination of plasticizers A2 and B1 would be preferred in those instances when a softer, more compliant polymer composition is desired.

The maximum shear stress and peak load values for each of the cured compositions on aluminum and glass were measured using the Lap Shear test described in ASTM test procedure C 961-01. These values are recorded in Table 2.

**TABLE 2**

| Plasticizers | Max. Shear Stress (Lb /in²) | | Peak Load (Lb.f) | |
|---|---|---|---|---|
| | Aluminum | Glass | Aluminum | Glass |
| A1, B2* | 3 | 3 | 51 | 50 |
| A2, B1 | 5 | 3 | 49 | 50 |
| A3, B1 | 3 | 3 | 56 | 64 |
| A4, B1 | 3 | 4 | 54 | 72 |

| | | | | |
|---|---|---|---|---|
| * - Evaluated for comparative purposes | | | | |

All of the failures resulted from cohesive splitting within the body of the polymer compositions and not from adhesion failure to the glass or aluminum substrate.

### Example 3

This example demonstrates the effect of TMPD dibenzoate content in the plasticizer on the stability of the composition described as part B of in the preceding example 2. A prior art plasticizer, the mixed isobutyl /benzylphthalyl ester referred to hereinbefore as TMPDIBBP, was also evaluated as a comparison.

Samples weighing approximately 60 grams of compositions containing the ingredients of component B in the preceding Example 2 were weighed into a centrifuge tube using a 4 point balance and the weight recorded. The tube was then centrifuged at 4000 rpm for 60min. The tube was then removed from the centrifuge and the separated liquid was removed from the surface of the sample using a Pasteur Pipette. The liquid was then weighed using a 4 point balance. The percent weight loss (based on total sample weight) was calculated and is recorded in Table 3.

**Table 3**

| **B Component Stability by Centrifuge** | | | |
|---|---|---|---|
| | **Viscosity of plasticizer (cps)** | **Weight % *TMPDDB** | **Weight % Plasticizer loss** |
| ****TMPDIBBP** | 920 | 0 | 19.60 |
| **TMPDB₁** | 400 | 84.5 | 28.94 |
| **TMPDB₂** | 537 | 87 | 20.63 |
| **TMPDB₃** | 680 | 90.5 | 19.60 |
| **TMPDB₄** | 794 | 93.4 | 18.92 |
| **TMPDB₅** | 909 | 96.2 | 21.74 |

| | | | |
|---|---|---|---|
| **TMPDB = Dibenzoate of 2,2,4-trimethyl 1,3-pentanediol* **2,2,4-trimethyl-1,3-pentanediol isobutyrate(1) benzylphthalate(3) *TMPDB₁₋₅* = *Various Benzoate Ester compositions containing stated amounts of TMPDB* | | | |

The data in Table 3 demonstrate the improved stability of the compositions containing an elevated dibenzoate concentration over the composition containing 84.5% dibenzoate.

## Claims

1. A two-part curable polymer composition comprising: a first part that includes a cross-linkable polymer; and a second part that includes a liquid benzoate ester composition comprising a dibenzoate of the general formula 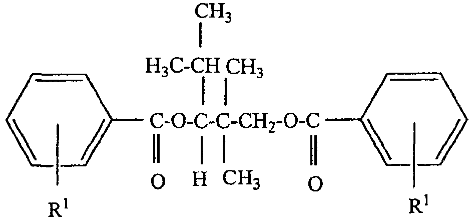 and at least one monobenzoate of a formula selected from the group consisting of 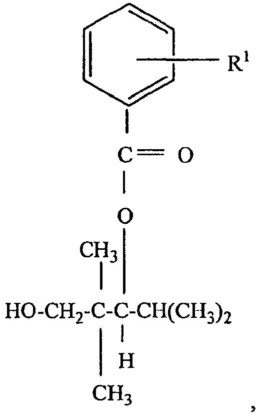 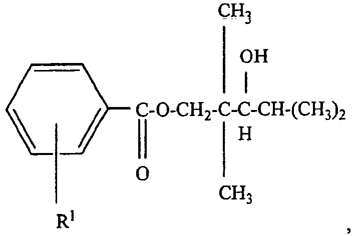 and mixtures thereof, wherein R¹ is alkyl or H, and the concentration of said diester is effective for imparting a viscosity of from 425 to 1200 centipoise (425 to 1200 mPa·s) measured at 25 °C using a Brookfield LVT viscometer, to said liquid benzoate ester composition, and a catalyst for cross-linking of the cross-linkable polymer;
wherein the concentration of said diester is above 87 weight percent of the benzoate ester composition.

2. The two-part polymer composition of claim 1 wherein said cross-linkable polymer is selected from the group consisting of polysulfides, polyurethanes, acrylic polymers, and mixtures thereof.

3. The two-part polymer composition of claim 2 wherein said polymer is a polysulfide elastomer.

4. The two-part polymer composition of claim 1 wherein the first part of said composition includes at least one plasticizer.

5. The two-part polymer composition of claim 4 wherein said plasticizer in the first part of said composition is selected from the group consisting of esters of benzoic acid and monohydric alcohols, esters of benzoic acid and diols, esters of benzoic acid with at least one glycol containing from 2 to 4 carbon atoms, esters of benzoic acid with oligomers of said glycol, and mixtures thereof.

6. The two-part polymer composition of claim 5 wherein the plasticizer in said first part is dipropylene glycol dibenzoate at a concentration effective for reducing the level of hardness and increase the level of pliability exhibited by said composition in the absence of said dibenzoate.

7. The two part composition of claim 5, wherein the benzoate ester composition includes from 0 to 5 weight percent of HOCH₂C(CH₃)₂CH(OH)CH(CH₃)CH₃, wherein the viscosity of the benzoate ester composition is at least 900 centipoise, when the benzoate ester composition includes said unesterified diol.

8. A method for increasing the strength of an insulating glass sealant comprising a polysulfide, a curing agent for said polysulfide and at least one plasticizer, said method comprising selecting said plasticizer from the group consisting of liquid benzoate ester compositions comprising a dibenzoate of the general formula 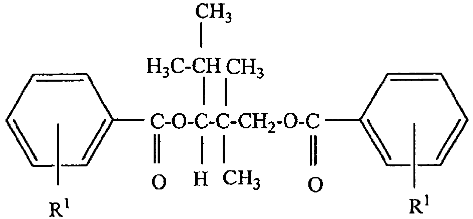 and at least one monobenzoate of a formula selected from the group consisting of 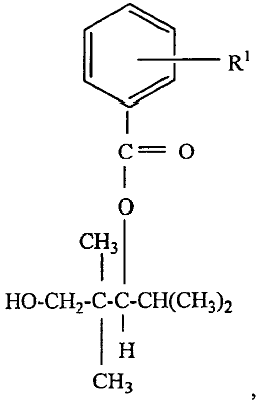 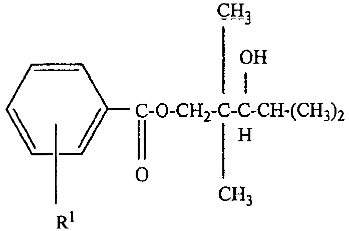 and mixtures thereof, wherein R¹ is alkyl or H, and mixtures thereof, the concentration of said diester being effective for imparting a viscosity of from 425 to 1200 centipoise (425 to 1200 mPa·s) measured at 25 °C using a Brookfield LVT viscometer, to said liquid benzoate ester composition;
wherein the concentration of said diester is above 87 weight percent of the benzoate ester composition.

9. A method according to claim 8 wherein a portion of said liquid benzoate ester composition is replaced with an equal weight of dipropylene glycol dibenzoate, said weight being effective for reducing the level of hardness and increase the level of pliability exhibited by said sealant as compared to a sealant where a portion of the benzoate ester is not replaced with glycol dibenzoate.

10. A method for increasing storage stability of a composition by reducing plasticizer separation, said method comprising selecting the plasticizer from the group consisting of liquid benzoate ester composition comprising a dibenzoate of the general formula 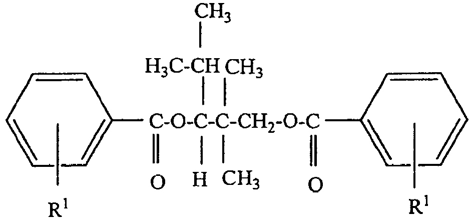 and at least one monobenzoate of a formula selected from the group consisting of 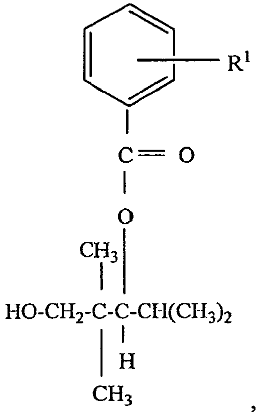 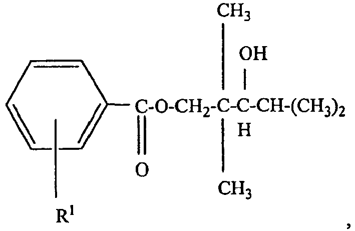 and mixtures thereof, wherein R¹ is CH₃ or H, the concentration of said diester is effective for imparting a viscosity of from 425 to 1200 entipoise (425 to 1200 mPa·s), measured at 25 °C using a Brookfield LVT viscometer, to said liquid benzoate ester composition; wherein the concentration of said diester is above 87 weight percent of the benzoate ester composition.

## Patentansprüche

1. Zweiteilige aushärtbare Polymerzusammensetzung umfassend: einen ersten Teil, der ein vernetzbares Polymer enthält; und einen zweiten Teil, der eine flüssige Benzosäureetester Zusammensetzung beinhaltet, umfassend einen Dibenzoesäureester der allgemeinen Formel 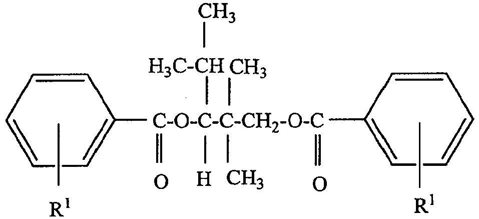 und wenigstens ein Monobenzoat einer Formel ausgewählt aus der Gruppe bestehend aus 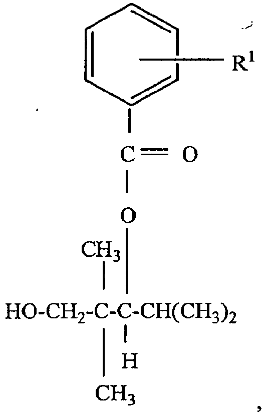 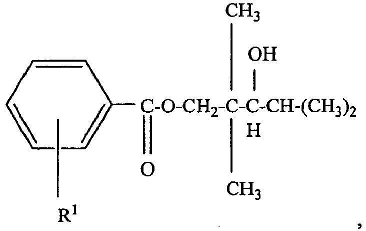 und Mischungen davon, wobei R¹ für Alkyl oder H ist, und die Konzentration des Diesters wirksam ist, eine Viskosität von 425 bis 1200 Centipoise zu verleihen, bei 25 ° C mit einem Brookfield-LVT Viskosimeter an der flüssigen Benzosäureester Zusammensetzung gemessen wird,
und einen Katalysator, um die das aushärtbare Polymer zu vernetzen;
wobei die Konzentration des Diesters oberhalb 87 Gewichtsprozent der Zusammensetzung liegt.

2. Zweiteilige Polymerzusammensetzung nach Anspruch 1, wobei das vernetzbare Polymer aus der Gruppe bestehend aus Polysulfiden, Polyurethanen, Acrylpolymeren und Mischungen davon ausgewählt ist.

3. Zweiteilige Polymerzusammensetzung nach Anspruch 2, wobei das Polymer ein Polysulfid-Elastomer ist.

4. Zweiteilige Polymerzusammensetzung nach Anspruch 1, wobei der erste Teil der Zusammensetzung mindestens einen Weichmacher beinhaltet.

5. Zweiteilige Polymerzusammensetzung nach Anspruch 4, wobei der Weichmacher in dem ersten Teil der Zusammensetzung aus der Gruppe ausgewählt ist bestehend aus Estern von Benzoesäure und einwertigen Alkoholen, Estern von Benzoesäure und Diolen, Estern der Benzoesäure mit mindestens ein Glycol mit 2 bis 4 C-Atomen, Ester der Benzoesäure mit Oligomeren des Glykols, und deren Mischungen.

6. Zweiteilige Polymerzusammensetzung nach Anspruch 5, wobei der Weichmacher im ersten Teil Dipropylenglykoldibenzoesäureester ist, in einer Konzentration, die wirksam ist den Grad der Härte zu reduzieren und den Grad der Biegsamkeit zu erhöhen, gegenüber einer entsprechenden Zusammensetzung, die keinen Dibenzosäureester enthält.

7. Zweiteilige Zusammensetzung nach Anspruch 5, wobei die Benzoesäureetester Zusammensetzung 0 bis 5 Gewichtsprozent HOCH₂C(CH₃)₂CH(OH)CH(CH3)CH3 enthält, wobei die Viskosität der Benzoesäureetester Zusammensetzung mindestens 900 Centipoise beträgt, wenn die Benzoesäureestester Zusammensetzung unverestertes Diol enthält.

8. Verfahren um die Widerstandskraft einer isolierenden Glasdichtungsmasse umfassend ein Polysulfid zu erhöhen, ein Härtungsmittel für das Polysulfid, und mindestens ein Weichmacher, wobei das Verfahren das Auswählen eines Weichmachers umfasst, ausgewählt aus der Gruppe bestehend aus flüssigem Benzoesäuretester Zusammensetzungen, die einen Dibenzoesäureester der allgemeinen Formel 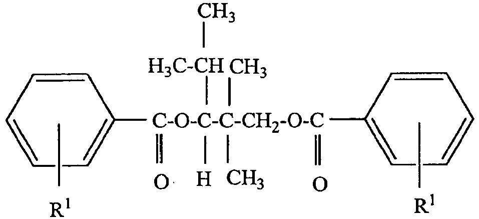 umfasst, und mindestens ein Monobenzoesäureester einer Formel ausgewählt aus der Gruppe, bestehend aus 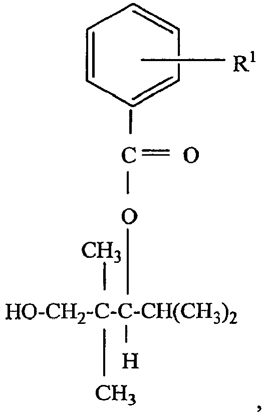 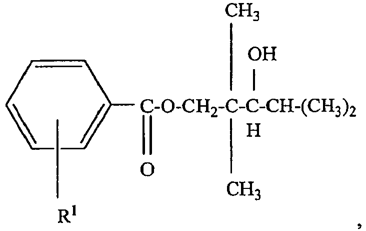 und Mischungen davon, wobei R¹ Alkyl oder H ist oder Mischungen und wobei die Konzentration des Diester wirksam ist eine Viskosität von 425 bis 1200 Centipoise zu vermitteln, gemessen bei 25 °C mit einem Brookfield-LVT Viskosimeter, an der flüssigen Benzosäureester Zusammensetzung; wobei die Konzentration des Diesters oberhalb 87 Gewichtsprozent der Zusammensetzung liegt.

9. Verfahren nach Anspruch 8, wobei ein Teil des flüssigen Benzoesäuretesters mit einem gleichen Gewicht von Dipropylenglykoldibenzoesäureester ersetzt wird, wobei das ersetzte Gewicht wirksam ist den Härtegrad der Dichtungsmasse zu verringern und den Grad der Biegsamkeit zu erhöhen, verglichen mit einer Dichtungsmasse der nicht ein Teil des Benzosäureesters mit Glykoldibenzoesäreester ersetzt wird.

10. Verfahren zur Steigerung der Lagerstabilität einer Zusammensetzung durch Verringerung der Weichmachertrennung, wobei das Verfahren das Auswählen des Weichmachers umfasst, ausgewähl aus der Gruppe bestehend aus einer flüssigem Benzoesäureester Zusammensetzung, umfassend ein Dibenzoesäureester der allgemeinen Formel 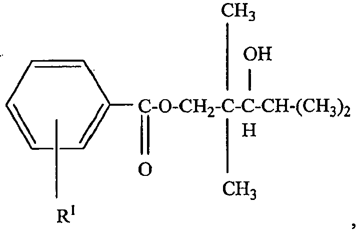 und mindestens einem Monobenzoesäureester einer Formel ausgewählt aus der Gruppe, bestehend aus 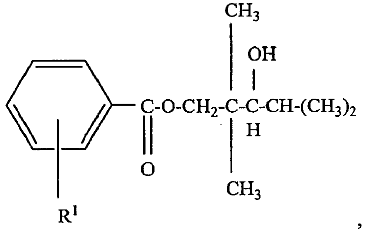 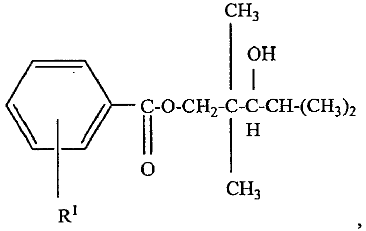 und Mischungen davon, wobei R¹ für CH₃ oder H steht, und wobei die Konzentration des Diesters wirksam ist eine Viskosität von 425 bis 1200 Centipoise zu verleihen, gemessen bei 25 °C mit einem Brookfield-LVT Viskosimeter an der flüssigen Zusammensetzung des Benzoesäureesters, wobei die Konzentration des Diesters oberhalb von 87 Gewichtsprozent der Zusammensetzung liegt.

## Revendications

1. Composition polymère durcissable en deux parties comprenant : une première partie qui comprend un polymère réticulable ; et une seconde partie qui comprend une composition liquide d'ester de benzoate comprenant un dibenzoate de la formule générale 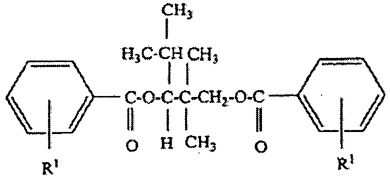 et au moins un monobenzoate d'une formule choisie dans le groupe constitué de 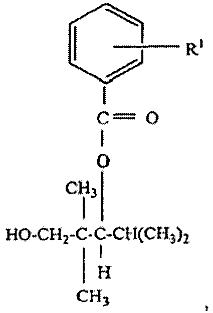 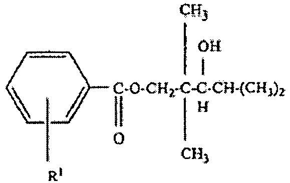 et de mélanges de ceux-ci, où R¹ est un groupe alkyle ou H, et des mélanges de ceux-ci, la concentration dudit diester étant efficace pour communiquer une viscosité de 425 à 1 200 centipoises (425 à 1 200 mPa.s), mesurée à 25°C en utilisant un viscosimètre Brookfield LVT, à ladite composition liquide d'ester de benzoate,
et un catalyseur pour la réticulation du polymère réticulable ;
dans laquelle la concentration dudit diester est supérieure à 87 pourcents en masse de la composition d'ester de benzoate.

2. Composition polymère en deux parties selon la revendication 1, dans laquelle ledit polymère réticulable est choisi dans le groupe constitué de polysulfures, polyuréthanes, polymères acryliques, et mélanges de ceux-ci.

3. Composition polymère en deux parties selon la revendication 2, dans laquelle ledit polymère est un élastomère de polysulfure.

4. Composition polymère en deux parties selon la revendication 1, dans laquelle la première partie de ladite composition comprend au moins un plastifiant.

5. Composition polymère en deux parties selon la revendication 4, dans laquelle ledit plastifiant dans la première partie de ladite composition est choisi dans le groupe constitué d'esters d'acide benzoïque et d'alcools monovalents, d'esters d'acide benzoïque et de diols, d'esters d'acide benzoïque avec au moins un glycol contenant de 2 à 4 atomes de carbone, d'esters d'acide benzoïque avec des oligomères dudit glycol, et de mélanges de ceux-ci.

6. Composition polymère en deux parties selon la revendication 5, dans laquelle le plastifiant dans ladite première partie est le dibenzoate de dipropylèneglycol à une concentration efficace pour réduire le niveau de dureté et augmenter le niveau de pliabilité présentés par ladite composition en l'absence dudit dibenzoate.

7. Composition en deux parties selon la revendication 5, dans laquelle la composition d'ester de benzoate comprend de 0 à 5 % en masse de HOCH₂C(CH₃)₂CH(OH)CH(CH₃)CH₃, dans laquelle la viscosité de la composition d'ester de benzoate est d'au moins 900 centipoises, lorsque la composition d'ester de benzoate comprend ledit diol non estérifié.

8. Procédé d'augmentation de la résistance d'un agent d'étanchéité de verre isolant comprenant un polysulfure, un agent durcissant pour ledit polysulfure et au moins un plastifiant, ledit procédé comprenant le choix dudit plastifiant dans le groupe constitué de compositions d'ester de benzoate liquides comprenant un dibenzoate de la formule générale 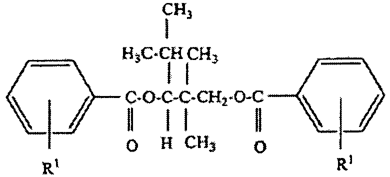 et au moins un monobenzoate d'une formule choisie dans le groupe constitué de 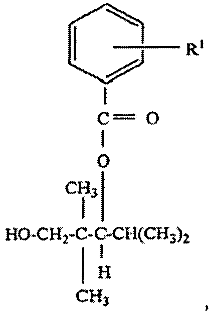 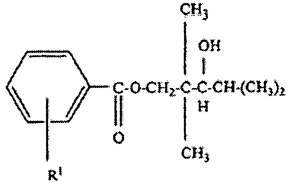 et de mélanges de ceux-ci, où R¹ est un groupe alkyle ou H, et des mélanges de ceux-ci, la concentration dudit diester étant efficace pour communiquer une viscosité de 425 à 1 200 centipoises (425 à 1 200 mPa.s), mesurée à 25°C en utilisant un viscosimètre Brookfield LVT, à ladite composition liquide d'ester de benzoate ;
dans lequel la concentration dudit diester est supérieure à 87 pourcents en masse de la composition d'ester de benzoate.

9. Procédé selon la revendication 8, dans lequel une portion de ladite composition liquide d'ester de benzoate est remplacée par une masse égale de dibenzoate de dipropylèneglycol, ladite masse étant efficace pour réduire le niveau de dureté et augmenter le niveau de pliabilité présentés par ledit agent d'étanchéité en comparaison avec un agent d'étanchéité dans lequel une portion de l'ester de benzoate n'est pas remplacée par du dibenzoate de glycol.

10. Procédé d'augmentation de la stabilité au stockage d'une composition par réduction de la séparation de plastifiant, ledit procédé comprenant le choix du plastifiant dans le groupe constitué d'une composition liquide d'ester de benzoate comprenant un dibenzoate de la formule générale 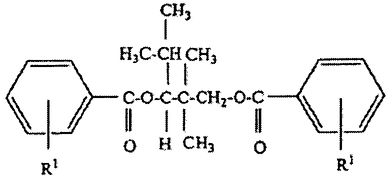 et au moins un monobenzoate d'une formule choisie dans le groupe constitué de 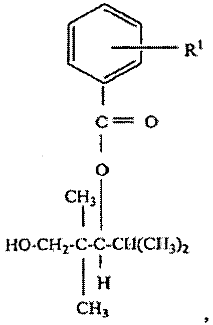 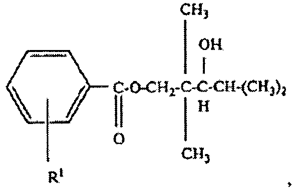 et de mélanges de ceux-ci, où R¹ est CH₃ ou H, la concentration dudit diester est efficace pour communiquer une viscosité de 425 à 1 200 centipoises (425 à 1 200 Mpa.s), mesurée à 25°C en utilisant un viscosimètre Brookfield LVT, à ladite composition liquide d'ester de benzoate ; dans lequel la concentration dudit diester est supérieure à 87 pourcents en masse de la composition d'ester de benzoate.
